# EUROPEAN PATENT SPECIFICATION

(11) **EP 3 454 177 B1**
(45) Date of publication and mention of the grant of the patent: **10.06.2020**
(21) Application number: 17190482.4
(22) Date of filing: 11.09.2017
(51) Int. Cl.: G06F 3/01, G06F 3/0482, G06F 3/0485, G06F 3/0484, G16H 40/63

(54) **METHOD AND SYSTEM FOR EFFICIENT GESTURE CONTROL OF EQUIPMENT**
VERFAHREN UND SYSTEM ZUR EFFIZIENTEN GESTENSTEUERUNG VON AUSRÜSTUNG
PROCÉDÉ ET SYSTÈME POUR UNE COMMANDE EFFICACE PAR GESTES D'UN ÉQUIPEMENT

(43) Date of publication of application: 13.03.2019
(73) Proprietor: Barco N.V., 8500 Kortrijk (BE)
(72) Inventor: SPALLA, Guillaume, 59110 La Madeleine (FR)
(74) Representative: IPLodge bv

(56) References cited:
- US-A1- 2013 271 397
- US-A1- 2016 216 768

## Description

The present invention relates to methods, systems and devices for interaction via a display-based user interface using gestures. The present invention particularly relates to environments in which extreme cleanliness, sterility, asepsis or biohazard avoidance is required such as surgical operation rooms and clean rooms.

### Background

The possibility for an operator of being able to control features in his/her environment with touchless interaction such as gestures, can be advantageous or even necessary.

In the field of healthcare it can for some applications be of importance to preserve sterile conditions. During e.g. a minimally invasive surgical operation, the main source of information for a surgeon is given by the endoscopic video stream, shown on a display. Thus the surgeon and his/her assistants are continuously looking at this display. Sometimes they need to switch to another video source, for instance showing ultrasound images, or to look at CT (Computed Tomography) images. Additionally, the operating team might need to adjust the light of the room, the music played in the room, or change the position of the patient bed.

The surgeons and their assistants manipulating the surgical tools or touching the patient have to stay in a so-called "sterile field", i.e. they cannot touch any non-sterile object, to avoid risking contaminating the patient. They even should not exit the sterile field with their hands, even in mid-air, i.e. they need to stay within an aseptic or sterile zone. Thus none of these OR personnel can interact with the surgeon display or with other devices in the room (light, music, bed position, phone calls etc). They need to ask a non-sterile operator, located outside the sterile field, to perform such action. It would be more efficient if the OR personnel could perform such operations themselves without having to leave the sterile field.

Gesture controlled displays are known in the art, however, a gesture controlled display system used in an operating room would need to fulfil the above requirements and at the same time be user friendly to the operator. For example it is important to minimize the risk that an arbitrary movement with the hand is considered to be an actual input gesture to the display (false positive) or that an actual gesture is not recognised (false negative).
US patent application US20160216768 A1 describes a method for a gesture controlled medical system where a start and stop gesture is provided, indicating the beginning and end of a gesture control mode. In some embodiments of the described method, the gesture control mode is exited based on one or more exit triggers to prevent the gesture control mode from remaining active for periods of time during which gesture input may be inadvertent. The exit triggers include determining that the duration of time in the gesture control mode exceeds a predetermined gesture control timeout, a predetermined progress of the medical procedure, or detecting unexpected objects in an enveloppe within which gestures can be detected.
US 2013/0271397 A1 discloses techniques for a method and apparatus for re-engagement of gesture control in a tracking system. An engagement gesture may include a user maintaining a particular pose for a pre-determined period of time in order to engage the tracking system. An engagement pose allows a gesture system to ignore hand motions that are not intended to be control gestures and hence can make a system more robust. After tracking the gesture over a period of time, the computer system may exit the mode of operation (gesture tracking mode and/or command mode). For example, the computer system exits the mode of operation after the user performs the user gesture. Once the user gesture is complete, the computer system may exit the gesture tracking mode and/ or command mode. At this point, the computer system may no longer accept a gesture input.

### Summary of the invention

It is an objective of the present invention to provide methods, systems and devices for interaction via a display-based user interface using gestures. Additionally, an object can be to improve usability and intuitiveness of human-machine interaction such as touchless human-machine interaction and bring the system to a working product. Another object can be to reduce the number of necessary interaction steps in human-machine systems such as hands-free human-machine systems. Another object can be to improve the quality or relevance of feedback to an operator.
The present invention particularly relates to environments in which extreme cleanliness or sterility or asepsis or contamination avoidance is required such as surgical operation rooms, clean rooms and biohazard rooms. Embodiments of the present invention can overcome one or more of the above mentioned deficiencies. Even though the exemplary embodiments of the present invention refer to a healthcare environment, the method steps and corresponding systems may as well provide a user friendly and secure way of working with gesture controls within other fields, e.g. in semiconductor processing clean rooms, USP 797 clean rooms, or biocontainments for isolation of dangerous biological agents in an enclosed laboratory facility.

The present invention comprises a solution enabling, for example touchless interaction via a display-based user interface considering any, some or all of the following features:
- The system can work particularly with small gestures (limlited range gestures, such as within a distance of 100cm) so that the operator can avoid leaving a sterile, aseptic or contamination-free field (preferably with his/her hands alone).
- The system can work together with a Graphical User Interface (GUI) shown on a display: The display can be the primary source of information for an operator (e.g. the surgeon) and is a logic point of interaction.
- The GUI can be non-intrusive to reduce or avoid hiding operator's tools such as an endoscope video stream.
- The GUI can be designed to be generic, i.e. to control heterogeneous functionalities beyond the display itself.
- Gestures and GUI can be designed for efficiency so that the extent or the number of efforts to trigger an action can be reduced, e.g. to the minimum.
- Interactions can be designed in such a way that the risk of false positives or false negatives can be reduced e.g. to a minimum.

In one embodiment of the present invention there is provided a method as defined in the appended independent claim 1. The method is for controlling a computer based system by using gestures; the computer based system comprises a controller, at least one computer based target application, and a gesture sensor, which is controlled by a computer based gesture sensor application. The computer based gesture sensor application can run on a computer system under the control of a processor and volatile and/or non-volatile memory, for example. The controller is connected with the computer based target application and the computer based gesture sensor application. The method comprises the steps of the computer based gesture sensor application being in an activation mode allowing the gesture sensor to receive n arbitrary input gestures, where n is greater than one. The gesture sensor then receives the arbitrary input gestures and the computer based gesture sensor application forwards them to the controller, the controller instructs the at least one computer based target application to adapt according to the arbitrary input gestures, and the controller then automatically assigns an idle mode to the computer based gesture sensor application. Idle mode can be a mode in which a gesture can be recognised to bring the idle mode into an activation mode but no other gesture will be recognised. This has the advantage of increasing user control and minimizing the risk of false positives, especially for the case of the controller only allowing a limited number of gestures before entering the idle mode.
Additionally or alternatively, the method can comprise the computer based system providing feedback after every change of mode of the computer based gesture sensor application, said feedback being visual or audible or haptic or any combination of the three.
This has the advantage of giving feedback to the operator during the process to inform which state the system is in. In particular the feedback can provide information to the operator as to whether a gesture has been correctly identified (avoidance of false positive or false negative).
Additionally or alternatively, the method can comprise the computer based system having a display with a display area and menu choices, and the computer based system can output the menu choices, e.g. in a part of the display such as at the edges of the display area. This has the advantage of making it easy for the operator to interact with- and enter the menus since they are in a known position that is easy to find, especially in the case of having one menu per display edge. Additionally the menus do not obscure important content in the centre of the display. The limited number of choices can allow the user to quickly select an option and/or to remember by heart which one he/she wants to select. Dedicated gestures can allow selection of the options without the need of using a mouse or other cursor like a pointing system, thus the distance to be moved to enable an option is minimal.

Additionally or alternatively, the method can comprise the computer based system having a display showing a sliding scale or a set of discrete ordered choices and a means for recognising performed gestures, so that the computer based system can initiate a move along the sliding scale or through the set of discrete ordered choices in a direction. This can be achieved by a dedicated gesture for this operation. This gesture can comprise a movement e.g. by a performed movement gesture which is recognised by the computer based system as moving from a first point to a second point in the same direction. The gesture movement is stopped and kept at the second point while the computer based system continues parsing through said scale or choices. Additionally, the speed of traverse of the movement gesture over the distance between the first and second point can be made proportional to the speed of the parsing. This has the advantage of providing a "virtual joystick" function where it's not necessary to exactly map the physical position of the gesture means (e.g. an operator hand) to a point on the display, but keeping the level indicator moving in the indicated direction. By simply adapting the position of the means for recognising or performing gestures, the parsing speed can be altered. Additionally or alternatively, the method can comprise the computer based system being adapted to recognise gestures or to have means for peforming gestures and a means for recognising such gestures, and the gesture sensor has an active field of detection, so that if a gesture is recognised or a means for performing gestures is sensed to be in proximity of a border of the active field of detection or of the display, e.g. within 0 to 20 percent of the area of field of view or range of the active field from a border of the active field of detection, the computer based system can provide a dedicated feedback, whereby said feedback can be visual or audible or haptic or any combination of the three.

This has the advantage that it can help the user to become aware of the system input boundaries.

Additionally or alternatively, the method can comprise the system being connected to devices or functions comprising any of a display, a networked system, a computer program product, room lighting, audio volume, electrical furniture or electronic communication devices, so that the controller can instruct the target application to adapt any settings of any of the above mentioned devices or functions.
Thus, the system does not have to be limited to e.g. display setting control, but could also control other features connected to the system, for example related to devices in the room of the operator (such as room lighting, motorized furniture, electronic communication devices, etc.).

In another embodiment of the invention there is provided a computer based gesture controlled system according to the appended independent claim 9. The system comprises a user interface, a gesture sensor, a computational unit comprising a processor such as a CPU and/or a GPU and volatile memeory such as a RAM and/or non-volatile memory, the computational unit running or executing a controller, a computer based gesture sensor application and at least one computer based target application. The gesture sensor is controlled by the computer based gesture sensor application, and the computer based gesture sensor application is connected to or co-operate with the controller, and the controller is connected to or co-operates with the at least one computer based target application. Additionally, the user interface can be display-based. The computer based gesture sensor application and the at least one computer based target application runs on a computer system under the control of the processor and a volatile and/or non-volatile memory.
Additionally or alternatively, the computer based system can comprise the gestures sensor being located on- or at the level of, the lower border of the display housing.
This has the advantage, in case the display is positioned so that its top-third part is on the same level as the eyes of an operator, and the operator is using his/her hands to perform the gestures, and if the gesture sensor is positioned at the lower border of the display housing, then the field of view of the gesture sensor will be optimally positioned for receiving the operator's gestures.

Additionally or alternatively, the computer based system can comprise or make use of any of, an "idle gesture" being an "open hand", an "activation gesture" being a "closed hand", a "confirmation gesture" being a "thumbs up", a "cancellation gesture" being a "thumbs down", an "input gesture" being a "swipe".

Additionally or alternatively, the computer based system can comprise or make use of a device which records a gesture and communicates the gesture to the gesture sensor application. The device can include accelerometers to record accelerations of the device when held in a hand and moved to create a gesture. The device can include a communication means such as a wireless or wired connection to a computer system outside the sterile zone. The wireless connection can be a radio frequency, infra-red, near field, optical, ultrasonic connection for example. The device can be configured to be cleaned and sterilised and may contain batteries that are charged via a non-invasive electromagnetic charging system.

### Brief description of drawings

Figure 1 shows a block diagram of an embodiment of the present invention comprising a display-based user interface for gesture control.
Figure 2 shows a block diagram of an embodiment of the present invention comprising a networked environment.
Figure 3 shows a block diagram of an embodiment of the present invention comprising a networked environment.
Figure 4 shows a block diagram of an embodiment of the present invention comprising a networked environment.
Figure 5a) to e) shows an examples of gestures used in an embodiment of the present invention.
Figure 6 shows a flow chart of an embodiment of the present invention.
Figure 7a) and b) shows menu choices in an embodiment of the present invention.

### Detailed description

### Definitions

A "computational unit" can be an electronic device having computing power and optionally network abilities. It can manage or run "applications" (e.g; as computer program products) that can control display content or display settings, or settings and functions of devices other than a display. A computational unit will typically be computer based. The computer may be standalone or the computational unit may be embedded in another device. A computational unit will typically include a processor and volatile and non-volatile memory as well as input/output ports such as at least one serial port, a communication interface, and input devices such as a mouse pointer and a keyboard.
An "application" can be a set of instructions to perform a task or a set of tasks implemented in a computer . The instructions can comprise moving data between different parts of the components in a computational unit e.g. between different parts of the computer such as between memory and a CPU or GPU. A "target application" is an application being controlled by the gesture controlled system. The target application can be a computer program running on a computer system, e.g. under the control of a processor. A "target application" when executed on a processor can comprise changing the settings of a "display" or a "room device".
A "display" can be an electronic device that presents information in visual form. It can be implemented with any of several techniques such as e.g. cathode-ray tube, or a fixed format display such as an LCD (liquid crystal display), LED (light emitting diode), OLED (organic LED), PDP (plasma display panel), etc. A display has a display area where content and/or a user interface can be displayed. A "room device" can be an arbitrary device in the room or location of an operator especially in a sterile environment. For example it can be a display, a networked system, a computer program product, a radio, tape recorder, cd player, wireless loudspeaker, room lighting device, motorized bed, telephone device, etc. The room device can be connected to the computational unit by cable or wireless, over a local network on-premise or over a global network e.g. over the internet.
A "gesture" can be a shape, posture or a movement of the body or parts of the body such as limbs of a person, hence a gesture can be in the form of a communication in which visible bodily actions or shapes communicate particular messages recognisable by a computer based system, either in place of, or in conjunction with, vocal or verbal communications such as speech. Although gestures can include movement of the hands, face, or other parts of the body, for example a person moving his/her hand, it also includes using other parts of the human body such as his/her vocal cords, or producing an EEG (Electroencephalography) signal and recognising patterns of EEG (Electroencephalography) signals in accordance with the present invention. Each intentional gesture should have distinguishing features so that one intentional gesture can be recognised and distinguished from another by a computer based system. The intentional gesture could be performed by or with an additional device, however it is preferred not to not bring additional devices into a protected e.g. sterile environment. Any additional suitable device that could be used should preferably be completely encased and be suitable for sterilisation at the level required for the protected e.g. sterile environment. If it contains batteries these should be charged via a non-invasive electromagnetic charging system and should not need removal from the sterile environment. The device may include accelerometers to recognise and record movements of hands or limbs of an operatior to create a gesture. It may include a means for communication such as a wired or wireless communication means, an Near Field Communication means, optical or RF communication means etc. "Gestures" described in the below embodiments are shown and described with reference to alternatively a left or right hand but this is for illustration purposes only. The invention is not limited hereto but each gesture can be performed with a left or a right hand according to the choice of the operator or by other means as indicated above.
A "gesture sensor" can be an electronic or electromagnetic wave or radiation sensor such as e.g. a visual camera e.g. a digital camera, or an infrared or ultraviolet camera, or an eye tracker or a brain sensor such as an EEG (Electroencephalography) sensor, or a scanning line sensor such as a scanning CCD line sensor or a depth camera such as a Time of Flight camera (TOF camera) or a seteroscopic camera system. A gesture sensor can record different gestures or set of gestures and it can be controlled by a "gesture sensor application" running on a computer system, e.g. under control of a processor. The gesture sensor application can run on a separate standalone computer device or can be embedded within the gesture sensor or in an external computational unit. A gesture sensor and a gesture sensor application can together be referred to as a "gesture sensor system".
A "menu" is a visible representation of a set of menu choices on a display area of a display. Each menu choice can have zero or more sub-menus in one or more sub-levels. If a menu choice has no sub-menu, an input signal, such as a gesture, can immediately activate a function.

### Embodiments

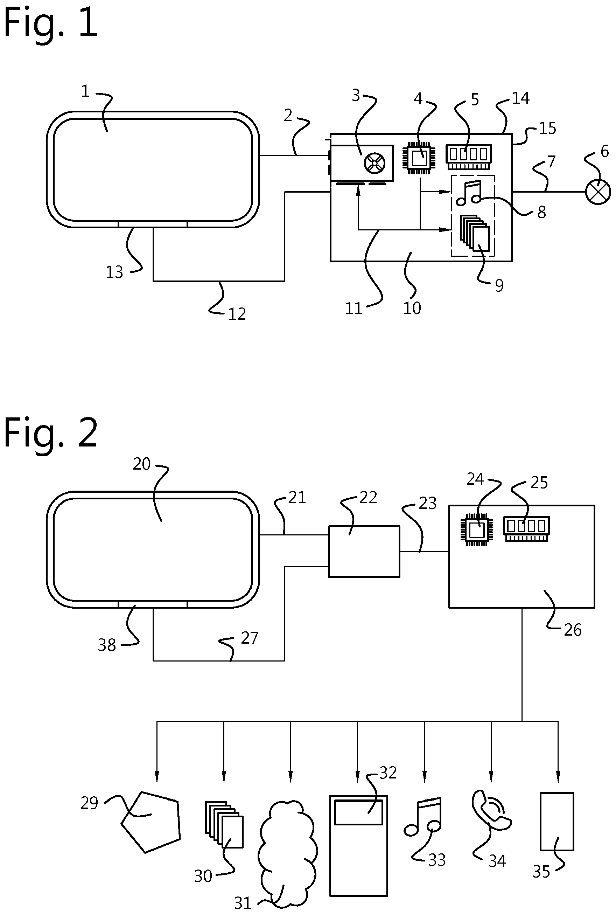

Figure 1 shows an embodiment of the present invention comprising a display 1 that can be connected to a computer system comprising a computational unit 10 via a video cable 2. The display can have a gesture sensor 13, for example a depth camera such as a digital camera, a Time of Flight camera or a stereoscopic camera system, being optionally embedded in the display housing. The gesture sensor 13 can also be attached onto or put next to the display housing (not shown)e.g. be a standalone device. The gesture sensor can be connected to the computational unit 10 via a wired connector such as a USB (Universal Serial Bus) connector 12 or a wireless connection e.g. RF, infrared, ultrasound, or optical communication means. The computational unit 10 can further comprise a processor such as a GPU (Graphics Processing Unit) 3, a processor such as a CPU (Central Processing Unit) 4, and a memory such as a non-volatile memory e.g. RAM (Random Access Memory) 5 and/or for example non-volatile memory. The GPU or CPU 4 can run, i.e. execute an application , e.g. a music control application 8, and/or an external light control application 6. The CPU 4 can execute possibly operating together with the GPU, an application 9, e.g. a CT (Computed Tomography) image browsing tool. The External Light control 6 can be connected with the computational tool with a cable such as a USB cable or with a wireless connection 7. The "external" applications 8 and 9 can be located in and executed by the computational unit as shown here, or remotely via a network connection.

The gesture sensor 13 and the gesture sensor application 14 (being an application implemented in a computer system comprising the computational unit 10) can be referred to as a gesture sensor system. The gesture sensor 13 can receive the gesture(s) and the gesture sensor application 14 can then forward the received gesture reguest to the controller 15, which in turn instructs the relevant target application to adapt accordingly.

The gesture sensor system can adopt or be assigned to different modes, e.g. "locked mode", "intermediate locked mode", "idle mode", "active mode" and "input mode". The way the gesture sensor system will react to a gesture depends on which mode the system has currently adopted or assigned to. The adoption of each mode can be controlled by a controller 15 implemented in the CPU 4, optionally the GPU 3 and the RAM 5 (which reside in the computational unit 10). The controller 15 can communicate with the gesture sensor 13 via the gesture sensor application 14 which is executed on the computer system.

Figure 2 shows another embodiment of the present invention comprising a display 20 that can be connected to a network video encoder/decoder 22 via a cable 21 or a wireless connection. The encoder/decoder 22 can be connected; for example to a computational unit 26 via an optical fibre 23.

The display can have a gesture sensor 38, for example a depth camera such as a digital camera, a TOF camera or a stereoscopic camera system, embedded in the display housing. The gesture sensor 38 can also be attached onto or put next to the display housing (not shown). The gesture sensor 38 can be a stand alone device. The gesture sensor 38 can be connected to the encoder/decoder 22 using wired connector such as a USB connector 27 or a wireless connection such as an optical, infra-red, ultraviolet, ultrasound or RF communication means. The computational unit 22 can comprise a processor such as a CPU 24, and a volatile memory such as RAM 25 and optionally non-volatile memory. The processor, e.g. CPU 24 can run e.g. execute several applications such as room lighting 29, e.g. operating room lamps, a database 30, e.g. a PACS database, a cloud service application 31 (e.g. a network video encoder/decoder management system), a motorized equipement, e.g. a motorized bed 32, music streaming equipment 33, a telephone 34 or any other remotely controlled device connected to the system 35.

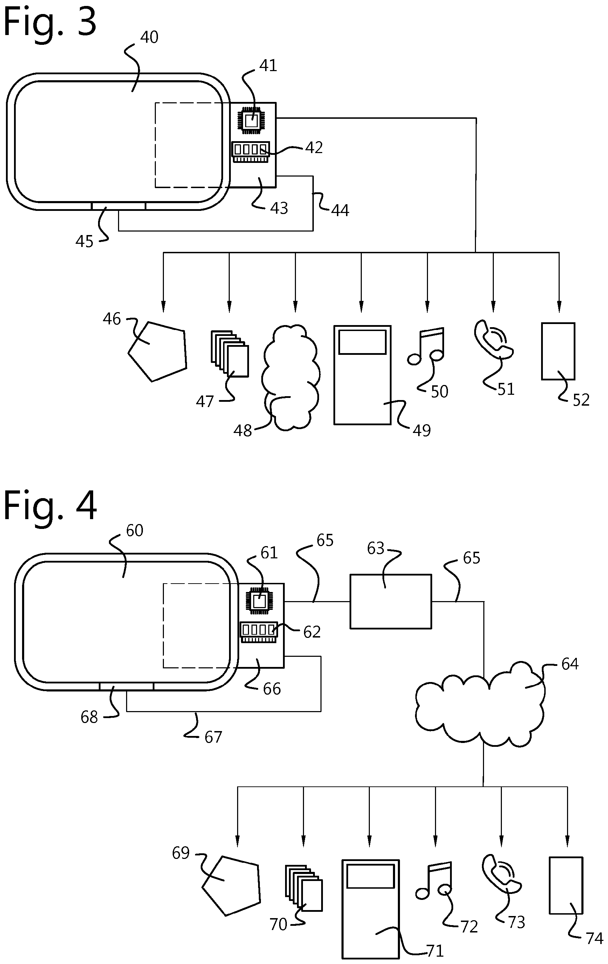

Figure 3 shows another embodiment of the present invention comprising a display 40 having a computational unit 43 embedded in the display 40. The computational unit 43 can comprise a processor such as a CPU 41 and a volatile memory RAM 42 and optionally a non-volatile memory. The processor e.g. CPU 24 can run several applications such as room lighting 46, e.g. operating room lamps, a database 47, e.g. a PACS database, a cloud service application 48 (e.g. a network video encoder/decoder management system), a motorized equipement, e.g. a motorized bed 49, music streaming equipment 50, a telephone 51 or any other remotely controlled device connected to the system 52.

Figure 4 shows another embodiment of the present invention comprising a display 60 having a computational unit 66 embedded in the display 60. The computational unit 66 can comprise a processor such as CPU 61 and a non-volatile memory such as a RAM 62 and optionally non-volatile memory, and it can be connected to a network video encoder/decoder 63 via an optical fibre 65 or other wired or wireless communication means. The encoder/decoder 62 can be connected to a network video encoder/decoder management system 64 via an optical fibre 65 or other wired or wireless communication means. The management system 64 can be connected to or co-operate with several applications running on a computer system such as a location specific lighting application 69, e.g. operating room lamps, a database application 70, e.g. a PACS database, or be connected to a motorized equipement, e.g. a motorized bed 71, music streaming equipment 72, a telephone 73 or any other remotely controlled device connected to the system 74. The applications 69 to 74 can be controlled by (e.g. executed by) a processor such as a CPU in the management system 64.
For healthcare applications that needs a sterile environment, it is preferred that the gestures are performed without having to bring additional devices into the sterile area. However, this is not a limitation of the present invention which could be implemented using an additional device to provide the gesture. A suitable device should preferably be completely encased and be suitable for sterilisation at the level required for the sterile room. If it contains batteries these should be charged via a non-invasive electromagnetic charging system. The device may include accelerometers to recognise and record movements of a gesture. It may include a means for communication such as a wired or wireless communication means.

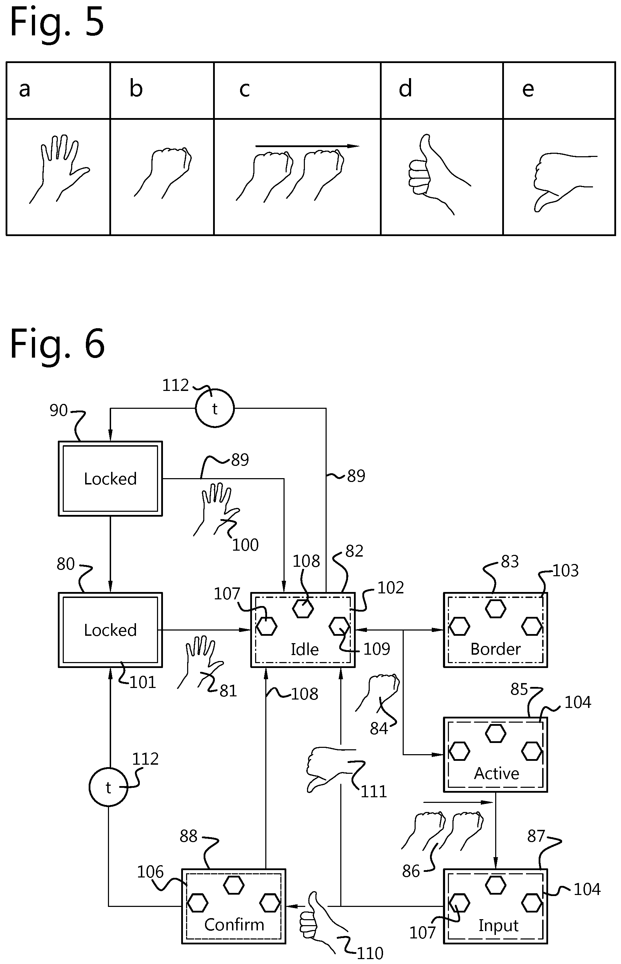

Figures 5 a) to d) show gestures of an embodiment of the present invention using a hand or the hands of the operator. The number of gestures comprised in the system of the present invention is preferably kept limited, in order to keep it simple for the operator to remember and carry out the gestures correctly. The gestures are shown from the perspective of the operator, who is looking at his/her hand, and the hand is positioned between the operator and the gesture sensor.

There can be an "idle gesture" e.g. an open hand as in figure 5a. If the gesture sensor system is in "locked mode" and receives an "idle gesture", it can adopt an unlocked or "idle mode" where it is ready to receive and interpret gestures from the operator. However it is preferred that when in idle mode a further gesture is required to bring the state to activation mode. There can be an "activation gesture" e.g. a closed hand as in figure 5b). The "activation gesture" can put the gesture sensor system into an "active mode" where it is ready to receive an "input gesture". The "input gesture" can be a "swipe" illustrated in figure 5c as a closed hand moving from left to right. Several directions of the swipe can be possible. Thus, the sequence "idle gesture", "activation gesture" and "input gesture" can be implemented with the gesture sequence "open hand", "closed hand" and "swipe" in figure 5a), b) and c) respectively. There can be a "confirmation gesture" and a "cancellation gesture" e.g. the "thumbs up" and "thumbs down" in figure 5d) and e) respectively. These gestures can be used to confirm or cancel an ongoing operation.
The gestures described above can be easy for the operator to physically combine into one continuous movement. Together with menu choices e.g. located at the border of the display area, the operator can activate a function of a menu choice by making a movement like grabbing it from the display border and bringing it to the center of the display. The inventor has found during trials with test persons that such work flows were perceived as having the advantages of being both ergonomic and intuitive.

In one embodiment outside the scope of the present invention the gesture sensor system is configured such that it will only accept one "input gesture" and will then automatically return to "idle mode". If the operator wants to perform another gesture, he/she has to make an "activation gesture" or an "idle gesture" and then again an "activation gesture" to prepare the system for an "input gesture". This work flow has the advantage that the operator does not risk to have one or more unintentional gestures accidentially interpreted as one or more "input gestures" when in the idle mde or after an input gesture has been recognised in the activation mode. Alternatively, giving a similar advantage, in the invention the gesture sensor system can accept a pre-defined number, greater than one, of intentional gestures before it automatically returns to "idle mode".

Between each/predefined number of "input gesture(s) the operator can make an "idle gesture" followed by an "activation gesture" to again prepare the system for one or more "input gesture(s)". The system can automatically return to "idle mode" after the pre-determined "intput gesture(s)" have been received. In combination with system feedback for each step confirming the current state of the gesture sensor system, the operator can maintain full control and overview of the process.

Figure 6 shows a flow chart of an embodiment of the present invention comprising a sequence of gesture interactions given by an operator to a display-based system which can be used with any of the system embodiments of the present invention. Initially there is a display area 80 and the gesture sensor system is in a locked mode, which can be confirmed by the screen having a "locked feedback border" 101. Other means of confirmation can be used, e.g. audio, optical or haptic feedback.
The performance and recognition of a dedicated gesture such as an "unlock gesture" or "idle gesture" 81 changes the gesture sensor system into an "idle mode", which can be confirmed in display area 82 showing the "idle feedback border" 102. Other means of confirmation can be used, e.g. audio, optical or haptic feedback. The menu, here with the menu choices 107, 108 and 109, can become visible in the idle mode, e.g. on the display or by other means. In general there is no limitation on the number of menu choices possible. However, more than one menu choice per display edge might make it more complicated to select a menu choice by the performance and recognition of a gesture.

If no further input is given (e.g. no hand is seen by the gesture sensor) during a time t 112, it is an option that the gesture sensor system can automatically go to an "intermediate locked mode", which can be confirmed by showing display area 90 via path 89. Other means of confirmation can be used, e.g. audio, optical or haptic feedback. The operator then can have the possibility to return to the previous menu (in this case the the idle mode showing the display area 82) by a further gesture such as by showing his hand e.g. in an "idle gesture" 100. This timeout to an "intermediate locked mode" can be accessible from any mode (but is only shown for the idle mode with display area 82 in this example). When the gesture sensor system is in the "intermediate locked mode", the user can again avoid giving input (e.g. by not showing his/her hand to the gesture sensor) for a time t 142, then the gesture sensor system can go into the initial "locked mode", which can be confirmed as shown by display area 80 being visible. Other means of confirmation can be used, e.g. audio, optical or haptic feedback.

In order to prepare the gesture sensor system for receiving input gestures, a dedicated gesture such as an "idle gesture" 136 can be performed and can be recognised by the gesture sensor system. The gesture sensor system can then be put in "active mode" by an activation gesture 84 which can be confirmed by the "active feedback border" 104 appearing on the display area 85. Other means of confirmation can be used, e.g. audio, optical or haptic feedback. The operator can now select a menu choice by making a dedicated gesture such as a swipe in the direction that corresponds to going from the edge where the menu is located to the middle of the screen, for example left to right for selecting menu 107. A sub-menu related to the chosen application may appear from which the operator can select (using a gesture or modifying a gesture) a value e.g. by halting a moving level indicator with an "idle gesture" 135. The operator can then choose bewteen selecting the current level value by the confirmation gesture "thumbs up" 110 or cancel the operation by cancellation gesture "thumbs down" 111. If the operator selects the confirmation gesture, e.g. "thumbs up" 110, which the gesture sensor system can recognise, the display system can provide a confirmation feedback 106 (or other means of optical, audio or haptic feedback can be used), or alternatively, the display system merely performs the requested action of the menu choice. In this case there is no feedback 106 shown. After the requested action has been implemented the system automatically returns 136 to "idle mode" 82. If the operator selects to cancel the operation e.g. with the "thumbs down gesture" 111, the system will return to "idle mode" 82 without implementing the menu choice.

In another embodiment of the present invention, if the menu choice has no sub-menu, for example it is a video, the menu choice can be immediately implemented after the swipe gesture has been recognised by the the gesture sensor system and the menu choice has been activated by it. For example, if menu choice 107 comprises a video, the operator activates it by making a gesture to bring it to the center of the display by a "swipe" 86. The video can then immediately be displayed on the display area, and the system can automatically return to "idle mode" 82. For healthcare applications it can be advantageous to have video switching easily accessible due to its frequent use. The menu choice of video content can be a displayed thumbnail comprising a static image or showing the actual video stream. This can facilitate operation procedures for the operator.

According to the present invention the display system and the gesture sensor system are adapted to receive more than one gesture as the pre-determined number of gestures before automatically implementing the command corresponding to the gesture. This has the advantage of reducing the risk of a false positive, i.e. registering an unintentional move by the operator as an input gesture. The operator knows how many gestures the system is expecting and can proceed accordingly. The inventor has however found that allowing only one gesture before automatic entry into idle mode significantly reduces the risk of a false positive and helps the operator to reduce stress (e.g. less worry he/she would provide unintentional gestures) and maintain control. The input gesture itself can be of arbitrary shape and form.

Display area 83 illustrates what can happen if a gesture has been positioned close to the edge, e.g. 10 % from the edge, i.e. within an edge area which has 10% of the area of the field of view of the gesture sensor. (In a general case this can mean that the operator input is approaching the limits of the detection means.) A "proximity feedback border" 103 can alert the operator that he/she is about to move out of the detectable field of the gesture sensor. Other means of alerting can be used, e.g. audio, optical or haptic feedback. This warning mode 83 can be activated from any non-locked mode. The situation can be resolved by simply moving the hand (or other gesture means) further away from the edge and the gesture sensor system can e.g. go back to "idle mode".

There are various alternative ways of how the display system can give feedback to the operator while he/she performs the gestures. Figure 6 refers to "feedback borders" in the display area overlayed onto the content already shown on the display. There can be other visual arrangements, such as light emitting diodes or other light sources, attached to the display housing or placed next to the display housing. Alternatively the system can provide audible signals or provide haptic feedback, e.g. in form of air pressure variations.

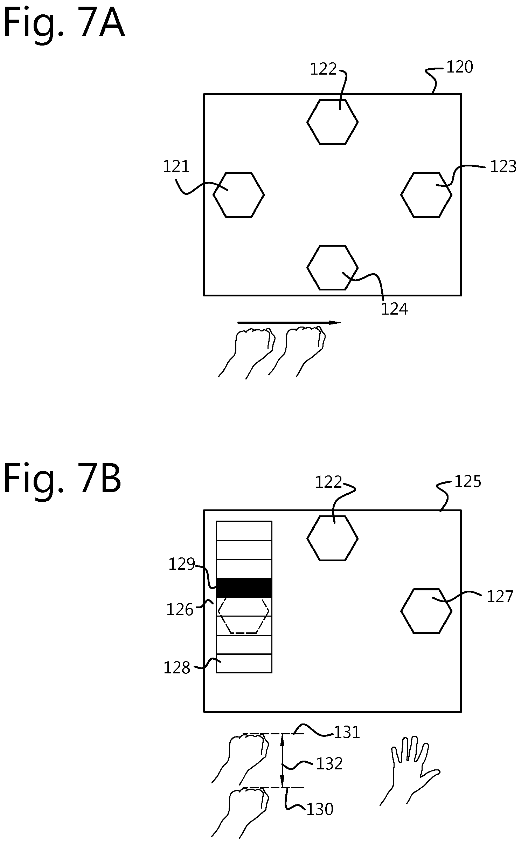

Figures 7a) and b) illustrate an embodiment of the present invention when menu choices are activated.
Figure 7a) shows a display area 120 where four menu choices 121, 122, 123 and 124 are visible. The operator can activate a menu choice 122 by performing a dedicated gesture such as a swipe gesture 139 in a vertical direction. The menu choice 122 can have one or more sub-menus, like a new menu e.g. with menu choices 126 and 127 can appear. Menu choice 126 (e.g. containing a sliding scale) can be activated by "bringing it to the center of the display" i.e. by making a swipe gesture from left to right 140. The sliding scale 128 can then appear with a current level indicator 129, as seen in figure 7b). Alternatively, when the sub-menu choice (here, the sliding scale 128) is displayed, the menu choices 121, 126 and 127 could be dimmed or completely hidden (not shown).

The operator can then move the level indicator 129 upwards along the sliding scale 128 by providing a further gesture such as a swipe gesture from an initial position 130 and upwards to a point 131 (thus, the gesture would be in the same direction as the move of the level ndicator). The relation between the gesture and the level indicator 129 can be of a "virtual joystick" character, i.e., the level indicator 129 will continue to move as long as the hand (or other gesture means) is kept at the distance 132. The operator can, for example increase or decrease the speed of the level indicator movement, on the fly, by increasing or decreasing distance 132 while the level indicator 129 is moving. When the level indicator 129 is positioned at a desired value, the operator can provide an "idle gesture" 141 to stop the movement (compare with 135 in figure 6).

Alternatively if the operator moves the (still closed) hand back to level 130 (but no further), the speed of the level indicator will be decreased. If the operator goes below the level 130 the level indicator 129 will start to move in the opposite direction. When the wanted value of the level indicator is finally reached, the operator opens his/her hand in an "idle gesture" 141 and the movement is stopped. The level indicator could also be stopped when it reaches the end of the scale. The operator can close his/her hand again to further move the slider, or do a "confirmation gesture" such as "thumbs up" in figure 4d). The "confirmation gesture" confirms that the current position of the level indicator should be used. (Compare with 110 and 111 in figure 6.) A menu choice can also comprise a "carousel" of discrete values that can be rotated, until an "idle gesture" is provided for stopping at a value. This might be suitable for e.g. parsing through a certain number of PACS (Picture Archiving and Communication System) data files.

The "virtual joystick" character gives the possibility for the gesture means, e.g. the operator hand, to not to be used as a "pointing device" to move the cursor on the screen. The inventor has found that a one-to-one correspondence between the gesture means (e.g. the hand) and a cursor on the display screen is difficult to manage since the movements have to be precise. The "virtual joystick" way of working provides more freedom for the operator to perform the gesture without compromising correct readings.

Computer based systems and methods as described with respect to embodiments of the present invention can include servers, display devices, back-end devices, rendering devices, streaming devices for example each optionally able to display images and videos and provide a selection menu. These computer based systems and methods can be performed by a standalone digital processing device or can be embedded in another device. Such devices or methods can use a processor i.e. a digital processing engine to carry out functions. The processing engine preferably has processing capability such as provided by one or more microprocessors, FPGA's, or a central processing unit (CPU) and/or a Graphics Processing Unit (GPU), and is adapted to carry out the respective functions by being programmed with software, i.e. one or more computer programmes. References to computer based systems and methods and software can encompass any type of programs in any language executable directly or indirectly by a processor, either via a compiled or interpretative language such as Java or Python. The implementation of any of the methods of the present invention can be performed by or assisted by logic circuits, electronic hardware, processors or circuitry which can encompass any kind of logic or analog circuitry, integrated to any degree, and not limited to general purpose processors, digital signal processors, ASICs, FPGAs, discrete components or transistor logic gates and similar.

Such devices may have memory (such as non-volatile memory, non-transitory computer readable medium, RAM and/or ROM), an operating system, optionally a display such as a fixed format display, ports for data entry devices such as a keyboard, a pointer device such as a "mouse", serial or parallel ports to communicate other devices, network cards and connections to connect to any of the networks.

The software can be embodied in a computer program product adapted to carry out the functions itemised below when the software is loaded onto the controller and executed on one or more processing engines such as microprocessors, ASIC's, FPGA's etc. devices for use with any of the embodiments of the present invention can incorporate a computer system capable of running one or more computer applications in the form of computer software.
The methods of embodiments of the present invention can be performed by one or more computer application programs such as the target application or the gesture sensor application running on the computer system by being loaded into a memory and run on or in association with an operating system such as Windows™ supplied by Microsoft Corp, USA, Linux, Android or similar. The computer system can include a main memory, preferably random access memory (RAM), and may also include a non-transitory hard disk drive and/or a removable non-transitory memory, and/or a non-transitory solid state memory. Non-transitory removable memory can be an optical disk such as a compact disc (CD-ROM or DVD-ROM), a magnetic tape, which is read by and written to by a suitable reader. The removable non-transitory memory can be a computer readable medium having stored therein computer software and/or data. The non-volatile storage memory can be used to store persistent information that should not be lost if the computer system is powered down. The application programs may use and store information in the non-volatile memory.
The software embodied in the computer program product is adapted to carry out any of the functions of the methods of the present disclosure and the methods of the attached claims when the software is loaded onto the respective device or devices and executed on one or more processing engines such as microprocessors, ASIC's, FPGA's etc, for example
performing a method for controlling a computer based system by using gestures, the computer based system comprising a controller, at least one computer based target application, and a gesture sensor controlled by a computer based gesture sensor application that can adopt different modes, the controller being connected with or co-operating with the at least one computer based target application and the computer based gesture sensor application.

The software embodied in the computer program product is adapted to carry out the following functions when the software is loaded onto the respective device or devices and executed on one or more processing engines such as microprocessors, ASIC's, FPGA's etc.:
performing a computer based gesture sensor application which can be placed in an activation mode allowing the gesture sensor to receive n arbitrary input gestures where n is at least one, and/or
the gesture sensor receiving the n arbitrary input gestures, the computer based gesture sensor application forwarding the at least one input gesture to the controller, and/or
the controller instructing the at least one computer based target application to adapt according to the at least one input gesture, the controller automatically assigning an idle mode to the computer based gesture sensor application.

The software embodied in the computer program product is adapted to carry out the following functions when the software is loaded onto the respective device or devices and executed on one or more processing engines such as microprocessors, ASIC's, FPGA's etc.:
if the computer based gesture sensor application is in an activation mode the gesture sensor only needs to receive one input gesture before the computer based gesture sensor application enters idle mode.

The software embodied in the computer program product is adapted to carry out the following functions when the software is loaded onto the respective device or devices and executed on one or more processing engines such as microprocessors, ASIC's, FPGA's etc.:
providing feedback after every change of mode of the computer based gesture sensor application, said feedback being visual or audible or haptic or any combination of the three.

The software embodied in the computer program product is adapted to carry out the following functions when the software is loaded onto the respective device or devices and executed on one or more processing engines such as microprocessors, ASIC's, FPGA's etc.:
the computer based system comprising a display having a display area and menu choices, and outputting the menu choices at the edges of the display area, and/or
outputting one menu choice per edge of the display area.

The software embodied in the computer program product is adapted to carry out the following functions when the software is loaded onto the respective device or devices and executed on one or more processing engines such as microprocessors, ASIC's, FPGA's etc.:
the computer based system comprising a display showing a sliding scale or a set of discrete ordered choices and a means for recognising performed gestures, further comprising initiating a move along the sliding scale or through the set of discrete ordered choices, in a direction by recognition of a moving gesture, the movement of the gesture being from a first point to a second point in the same direction, then recognising that the gesture is kept still at the second point while parsing through said scale or choices continue, or
the distance between the first and second point is related to the speed with which the level indicator moves.

The software embodied in the computer program product is adapted to carry out the following functions when the software is loaded onto the respective device or devices and executed on one or more processing engines such as microprocessors, ASIC's, FPGA's etc.:
the sensor having an active field of detection, and if a performed gesture is sensed within an area adjacent to a border of the active field of detection, the area being 0 to 20 percent of the area of active field of detection, the system provides a dedicated feeback, said feedback being visual or audible or haptic or any combination of the three.

The software embodied in the computer program product is adapted to carry out the following functions when the software is loaded onto the respective device or devices and executed on one or more processing engines such as microprocessors, ASIC's, FPGA's etc.:
the system being connected to or cooperating with devices or functions comprising any of a display, a networked system, a computer program product, room lighting, audio volume, electrical furniture or electronic communication devices, and the method comprising the controller instructing the target application to adapt any settings of any of the above mentioned devices or functions.

The software embodied in the computer program product is adapted to carry out the following functions when the software is loaded onto the respective device or devices and executed on one or more processing engines such as microprocessors, ASIC's, FPGA's etc.:
feedback is provided after every change of mode of the computer based gesture sensor application, the feedback being visual or audible or haptic or any combination of the three.

The software embodied in the computer program product is adapted to carry out the following functions when the software is loaded onto the respective device or devices and executed on one or more processing engines such as microprocessors, ASIC's, FPGA's etc.:
recognisition of any of the gestures: the "idle gesture" being an "open hand", the "activation gesture" being a "closed hand", a "confirmation gesture" being a "thumbs up", a "cancellation gesture" being a "thumbs down", an "input gesture" being a "swipe".

Any of the above software may be implemented as a computer program product which has been compiled for a processing engine in any of the servers or nodes of the network or is written in an interpretive language such as Java that runs on a virtual machine such as the Java virtual machine. The computer program product may be stored on a non-transitory signal storage medium such as an optical disk (CD-ROM or DVD-ROM), a digital magnetic tape, a magnetic disk, a solid state memory such as a USB flash memory, a ROM, etc.

## Claims

1. A method for controlling a computer based system by using gestures, the computer based system comprising a controller (15), at least one computer based target application (6, 8, 9), means for recognising performed gestures, and a gesture sensor (13) controlled by a computer based gesture sensor application (14) that is configured to adopt different modes, the controller being connected with or co-operating with the at least one computer based target application and the computer based gesture sensor application, the method comprising the steps of :
the computer based gesture sensor application being in an idle mode and being brought into an activation mode in response to the gesture sensor receiving an activation gesture (84), the idle mode being a mode in which only the activation gesture is recognised and
the activation mode allowing the gesture sensor to receive a predefined number of arbitrary input gestures (86),
the gesture sensor receiving the predefined number of arbitrary input gestures, the computer based gesture sensor application forwarding the predefined number of arbitrary input gestures to the controller, the controller instructing the at least one computer based target application to adapt according to the predefined number of arbitrary input gestures,
and the controller then automatically assigning the idle mode to the computer based gesture sensor application,
**characterised in that** the predefined number is greater than one.

2. A method according to claim 1, comprising providing feedback after every change of mode of the computer based gesture sensor application, said feedback being visual or audible or haptic or any combination of the three.

3. A method according to any of the claims 1 to 2, wherein the computer based system comprises a display (1) having a display area (120) and menu choices (121-124), the method comprising outputting the menu choices at the edges of the display area.

4. A method according to claim 3, comprising outputting one menu choice per edge of the display area.

5. A method according to any of the claims 1 to 4, wherein the computer based system comprises a display (1) showing a sliding scale (128) or a set of discrete ordered choices, the method comprising initiating a move of a level indicator (129) along the sliding scale or through the set of discrete ordered choices, in a direction by recognition of a moving gesture, the movement of the gesture being from a first point (130) to a second point (131) in the same direction, then recognising that the gesture is kept still at the second point while parsing through said scale or choices continues.

6. A method according to claim 5, wherein the distance (132) between the first and second points is related to the speed with which the level indicator moves.

7. A method according to any of the claims 1 to 6, wherein the sensor has an active field of detection, and if a performed gesture is sensed within an area adjacent to a border of the active field of detection, the area being 0 to 20 percent of the area of active field of detection, the system provides a dedicated feeback, said feedback being visual or audible or haptic or any combination of the three.

8. A method according to any of the claims 1 to 7, wherein the system is connected to or cooperates with devices or functions comprising any of a display, a networked system, a computer program product, room lighting, audio volume, electrical furniture or electronic communication devices, and the method comprising the controller instructing the target application to adapt any settings of any of the above mentioned devices or functions.

9. A gesture controlled system having a user interface, a gesture sensor (13), means for recognising a performed gesture, a computational unit (10) comprising a processor (4) executing a controller (15), a computer based gesture sensor application (14) that is configured to adopt different modes, and at least one computer based target application (6, 8, 9), the gesture sensor being controlled by the computer based gesture sensor application, the computer based gesture sensor application being connected to or co-operating with the controller, the controller being connected to or co-operating with the at least one computer based target application, the computer based gesture sensor application being configured to have an idle mode and the gesture sensor being adapted to receive an activation gesture (84) to bring the computer based gesture sensor application into an activation mode, the idle mode being a mode in which only the activation gesture is recognised and the activation mode allowing the gesture sensor to receive a predefined number of arbitrary input gestures (86), the gesture sensor being configured to receive the predefined number of arbitrary input gestures, the computer based gesture sensor application being configured to forward the predefined number of arbitrary input gestures to the controller, the controller being configured to instruct the at least one computer based target application to adapt according to the predefined number of arbitrary input gestures, the controller being configured to then automatically assign the idle mode to the computer based gesture sensor application, **characterised in that** the predefined number is greater than one.

10. A gesture controlled system according to claim 9, wherein the user interface is display-based.

11. A gesture controlled system according to any of claims 9 and 10, wherein the gesture sensor is located on- or at the level of, a lower border of the display housing.

12. A gesture controlled system according to any of claims 9 to 11, comprising means for providing feedback after every change of mode of the computer based gesture sensor application, said means for providing feedback being visual or audible or haptic or any combination of the three.

13. A system according to any of claims 9 to 11, the "activation gesture" being a "closed hand", and an "input gesture" being a "swipe".

## Patentansprüche

1. Verfahren zum Steuern eines computergestützten Systems unter Verwendung von Gesten, wobei das computergestützte System eine Steuerung (15), wenigstens eine computergestützte Zielanwendung (6, 8, 9), Mittel zum Erkennen ausgeführter Gesten und einen Gestensensor (13) umfasst, der von einer computergestützten Gestensensoranwendung (14) gesteuert wird, die konfiguriert ist, verschiedene Modi anzunehmen, wobei die Steuerung mit der wenigstens einen computergestützten Zielanwendung und der computergestützten Gestensensoranwendung verbunden ist oder mit dieser zusammenarbeitet, wobei das Verfahren die Schritte umfasst:
wobei sich die computergestützte Gestensensoranwendung in einem Leerlaufmodus befindet und in einen Aktivierungsmodus gebracht wird in Reaktion wenn der Gestensensor eine Aktivierungsgeste (84) empfängt, wobei der Leerlaufmodus ein Modus ist, in dem nur die Aktivierungsgeste erkannt wird und wobei der Aktivierungsmodus es den Gestensensor ermöglicht, eine vordefinierte Anzahl von beliebigen Eingabegesten (86) zu empfangen,
das Empfangen des Gestensensors der vordefinierten Anzahl von beliebigen Eingabegesten, wobei die computergestützte Gestensensoranwendung die vordefinierte Anzahl von beliebigen Eingabegesten an die Steuerung weiterleitet,
wobei die Steuerung die wenigstens eine computergestützte Zielanwendung anweist, sich entsprechend der vordefinierten Anzahl beliebiger Eingabegesten anzupassen, und wobei dann die Steuerung automatisch der computergestützten Gestensensoranwendung den Leerlaufmodus zuweist,
**dadurch gekennzeichnet, dass** die vordefinierte Anzahl größer ist als eins.

2. Verfahren nach Anspruch 1,
umfassend das Bereitstellen von Rückmeldung nach jedem Moduswechsel der computergestützten Gestensensoranwendung, wobei die Rückmeldung visuell oder hörbar oder haptisch oder eine beliebige Kombination der drei ist.

3. Verfahren nach einem der Ansprüche 1 bis 2,
wobei das computergestützte System eine Anzeige (1) mit einem Anzeigebereich (120) und mit Menüauswahlmöglichkeiten (121-124) aufweist,
wobei das Verfahren die Ausgabe der Menüauswahlmöglichkeiten an den Kanten des Anzeigebereichs umfasst.

4. Verfahren nach Anspruch 3, umfassend das Ausgeben einer Menüauswahl pro Kante des Anzeigebereichs.

5. Verfahren nach einem der Ansprüche 1 bis 4, wobei das computergestützte System eine Anzeige (1) umfasst, die eine Gleitskala (128) oder einen Satz von einzelnen, geordneten Auswahlmöglichkeiten zeigt,
wobei das Verfahren das Initiieren einer Bewegung einer Niveauanzeige (129) entlang der Gleitskala oder durch der Satz der einzelnen, geordneten Auswahlmöglichkeiten in einer Richtung durch Erkennen einer sich bewegenden Geste umfasst, wobei die Bewegung der Geste von einem ersten Punkt (130) zu einem zweiten Punkt (131) in der gleichen Richtung ausgeht, dann das Erkennen umfasst, dass die Geste am zweiten Punkt still gehalten wird, während das Parsen durch die Skala oder die Auswahlmöglichkeiten fortgesetzt wird.

6. Verfahren nach Anspruch 5, wobei der Abstand (132) zwischen dem ersten und dem zweiten Punkt mit der Geschwindigkeit zusammenhängt, mit der sich die Niveauanzeige bewegt.

7. Verfahren nach einem der Ansprüche 1 bis 6, wobei der Sensor ein aktives Detektionsfeld hat und, wenn eine ausgeführte Geste in einem Bereich neben einem Rand des aktiven Detektionsfeldes erfasst wird, wobei die Fläche 0 bis 20 Prozent der Fläche des aktiven Detektionsfeldes beträgt, das System eine dedizierte Rückmeldung liefert, wobei die Rückmeldung visuell oder hörbar oder haptisch oder eine beliebige Kombination der drei ist.

8. Verfahren nach einem der Ansprüche 1 bis 7, wobei das System mit Vorrichtungen oder Funktionen verbunden ist oder mit diesen zusammenarbeitet, die ein(e)(en) beliebige(s)(n) einer Anzeige, eines vernetztes Systems, eines Computerprogrammprodukts, einer Raumbeleuchtung, einer Lautstärke, eines elektrischen Zubehör oder elektronischer Kommunikationsgeräte aufweisen, und wobei das Verfahren die Steuerung umfasst, welche die Zielanwendung anweist, beliebige Einstellungen einer beliebigen der oben genannten Vorrichtungen oder Funktionen anzupassen.

9. Gestengesteuertes System mit einer Benutzerschnittstelle, mit einem Gestensensor (13), mit Mitteln zum Erkennen einer ausgeführten Geste, mit einer Recheneinheit (10), die einen Prozessor (4) aufweist, der eine Steuerung (15) ausführt, mit einer computergestützten Gestensensoranwendung (14), die so konfiguriert ist, dass sie verschiedene Modi annimmt, und mit wenigstens einer computergestützten Zielanwendung (6, 8, 9),
wobei der Gestensensor von der computergestützten Gestensensoranwendung gesteuert wird, wobei die computergestützte Gestensensoranwendung mit der Steuerung verbunden ist oder mit dieser zusammenarbeitet, wobei die Steuerung mit der wenigstens einen computergestützten Zielanwendung verbunden ist oder mit dieser zusammenarbeitet, wobei die computergestützte Gestensensoranwendung konfiguriert ist, um einen Leerlaufmodus aufzuweisen, und der Gestensensor angepasst ist, um eine Aktivierungsgeste (84) zu empfangen, um die computergestützte Gestensensoranwendung in einen Aktivierungsmodus zu bringen, wobei der Leerlaufmodus ein Modus ist, in dem nur die Aktivierungsgeste erkannt wird, und der Aktivierungsmodus es dem Gestensensor ermöglicht, eine vordefinierte Anzahl von beliebigen Eingabegesten (86) zu empfangen,
wobei der Gestensensor so konfiguriert ist, um die vordefinierte Anzahl von beliebigen Eingabegesten zu empfangen, wobei die computergestützte Gestensensoranwendung so konfiguriert ist, um die vordefinierte Anzahl von beliebigen Eingabegesten zur Steuerung weiterzuleiten, wobei die Steuerung konfiguriert ist, um die wenigstens eine computergestützte Zielanwendung anzuweisen, sich entsprechend der vordefinierten Anzahl von beliebigen Eingabegesten anzupassen, wobei die Steuerung konfiguriert ist, um dann automatisch den Leerlaufmodus der computergestützten Gestensensoranwendung zuzuweisen,
**dadurch gekennzeichnet, dass** die vordefinierte Anzahl größer ist als eins.

10. Gestengesteuertes System nach Anspruch 9, wobei die Benutzerschnittstelle anzeigebasiert ist.

11. Gestengesteuertes System nach einem der Ansprüche 9 und 10,
wobei der Gestensensor auf oder auf der Höhe von einem unteren Rand des Displaygehäuses angeordnet ist.

12. Gestengesteuertes System nach einem der Ansprüche 9 bis 11, aufweisend Mittel zum Bereitstellen von Rückmeldungen nach jeder Änderung des Modus der computergestützten Gestensensoranwendung, wobei die Mittel zum Bereitstellen von Rückmeldungen visuell oder hörbar oder haptisch oder eine beliebige Kombination der drei sind.

13. System nach einem der Ansprüche 9 bis 11, wobei die "Aktivierungsgeste" eine "geschlossene Hand" ist und eine "Eingabegeste" ein "Wischen" ist.

## Revendications

1. Procédé pour commander un système informatisé en utilisant des gestes, le système informatisé comprenant une unité de commande (15), au moins une application cible informatisée (6, 8, 9), un moyen pour reconnaître des gestes effectués, et un capteur de gestes (13) commandé par une application pour capteur de gestes informatisée (14) qui est configurée pour adopter différents modes, l'unité de commande étant reliée à ou coopérant avec l'au moins une application cible informatisée et l'application pour capteur de gestes informatisée, le procédé comprenant les étapes suivantes :
l'application pour capteur de gestes informatisée étant dans un mode veille et étant amenée dans un mode d'activation en réponse au capteur de gestes recevant un geste d'activation (84),
le mode veille étant un mode dans lequel seul le geste d'activation est reconnu et le mode d'activation permettant au capteur de gestes de recevoir un nombre prédéfini de gestes d'entrée arbitraires (86),
le capteur de gestes recevant le nombre prédéfini de gestes d'entrée arbitraires, l'application pour capteur de gestes informatisée faisant suivre le nombre prédéfini de gestes d'entrée arbitraires à l'unité de commande, l'unité de commande ordonnant à l'au moins une application cible informatisée de s'adapter selon le nombre prédéfini de gestes d'entrée arbitraires,
et l'unité de commande attribuant alors automatiquement le mode veille à l'application pour capteur de gestes informatisée,
**caractérisé en ce que** le nombre prédéfini est supérieur à un.

2. Procédé selon la revendication 1, comprenant la fourniture de rétroaction après chaque changement de mode de l'application pour capteur de gestes informatisée, ladite rétroaction étant visuelle ou audible ou haptique ou n'importe quelle combinaison des trois.

3. Procédé selon l'une quelconque des revendications 1 à 2, dans lequel le système informatisé comprend un afficheur (1) ayant une zone d'affichage (120) et des choix de menu (121-124),
le procédé comprenant la sortie des choix de menu au niveau des bords de la zone d'affichage.

4. Procédé selon la revendication 3, comprenant la sortie d'un choix de menu par bord de la zone d'affichage.

5. Procédé selon l'une quelconque des revendications 1 à 4, dans lequel le système informatisé comprend un afficheur (1) présentant une échelle mobile (128) ou un ensemble de choix ordonnés discrets,
le procédé comprenant l'amorçage d'un mouvement d'un indicateur de niveau (129) le long de l'échelle mobile ou par l'intermédiaire de l'ensemble de choix ordonnés discrets, dans une direction par reconnaissance d'un geste de déplacement, le mouvement du geste allant d'un premier point (130) à un second point (131) dans la même direction, en reconnaissant ensuite que le geste est maintenu immobile au niveau du second point tandis que de l'analyse par décomposition par l'intermédiaire de ladite échelle ou desdits choix continue.

6. Procédé selon la revendication 5, dans lequel la distance (132) entre les premier et second points est liée à la vitesse avec laquelle l'indicateur de niveau se déplace.

7. Procédé selon l'une quelconque des revendications 1 à 6, dans lequel le capteur a un champ de détection actif, et si un geste effectué est détecté à l'intérieur d'une zone adjacente à une frontière du champ de détection actif, la zone faisant de 0 à 20 pour cent de la zone de champ de détection actif, le système fournit une rétroaction dédiée, ladite rétroaction étant visuelle ou audible ou haptique ou n'importe quelle combinaison des trois.

8. Procédé selon l'une quelconque des revendications 1 à 7, dans lequel le système est relié à ou coopère avec des fonctions ou des dispositifs comprenant n'importe lequel d'un afficheur, d'un système en réseau, d'un produit formant programme informatique, d'un éclairage de pièce, d'un volume audio, d'accessoires électriques ou de dispositifs de communication électroniques, et le procédé comprenant l'unité de commande ordonnant à l'application cible de s'adapter à n'importe quels paramétrages de n'importe lequel des dispositifs ou fonctions mentionnés ci-dessus.

9. Système commandé par gestes ayant une interface utilisateur, un capteur de gestes (13), un moyen pour reconnaître un geste effectué, une unité informatique (10) comprenant un processeur (4) exécutant une unité de commande (15), une application pour capteur de gestes informatisée (14) qui est configurée pour adopter différents modes, et au moins une application cible informatisée (6, 8, 9), le capteur de gestes étant commandé par l'application pour capteur de gestes informatisée, l'application pour capteur de gestes informatisée étant reliée à ou coopérant avec l'unité de commande, l'unité de commande étant reliée à ou coopérant avec l'au moins une application cible informatisée, l'application pour capteur de gestes informatisée étant configurée pour avoir un mode veille et le capteur de gestes étant conçu pour recevoir un geste d'activation (84) pour amener l'application pour capteur de gestes informatisée dans un mode d'activation, le mode veille étant un mode dans lequel seul le geste d'activation est reconnu et le mode d'activation permettant au capteur de gestes de recevoir un nombre prédéfini de gestes d'entrée arbitraires (86),
le capteur de gestes étant configuré pour recevoir le nombre prédéfini de gestes d'entrée arbitraires, l'application pour capteur de gestes informatisée étant configurée pour faire suivre le nombre prédéfini de gestes d'entrée arbitraires à l'unité de commande, l'unité de commande étant configurée pour ordonner à l'au moins une application cible informatisée de s'adapter selon le nombre prédéfini de gestes d'entrée arbitraires, l'unité de commande étant configurée pour attribuer alors automatiquement le mode veille à l'application pour capteur de gestes informatisée,
**caractérisé en ce que** le nombre prédéfini est supérieur à un.

10. Système commandé par gestes selon la revendication 9, dans lequel l'interface utilisateur est basée sur un afficheur.

11. Système commandé par gestes selon l'une quelconque des revendications 9 et 10,
dans lequel le capteur de gestes est situé sur ou au niveau d'une frontière inférieure du logement d'afficheur.

12. Système commandé par gestes selon l'une quelconque des revendications 9 à 11,
comprenant un moyen pour fournir de la rétroaction après chaque changement de mode de l'application pour capteur de gestes informatisée, ledit moyen pour fournir de la rétroaction étant visuel ou audible ou haptique ou n'importe quelle combinaison des trois.

13. Système selon l'une quelconque des revendications 9 à 11, le «geste d'activation » étant une « main fermée », et un « geste d'entrée » étant un « glissement ».
